# FASCICULE DE BREVET EUROPEEN

(11) **EP 0 833 573 B1**
(45) Date de publication et mention de la délivrance du brevet: **13.03.2002**
(21) Numéro de dépôt: 96922961.6
(22) Date de dépôt: 20.06.1996
(51) Int. Cl.: A23P 1/04, A23L 1/22

(54) **VEHICULES DE PRINCIPES ACTIFS A BASE DE TENSIOACTIFS NON IONIQUES ET LEURS APPLICATIONS NOTAMMENT DANS LES DOMAINES ALIMENTAIRE, COSMETIQUE ET PHARMACEUTIQUE**
WIRKSTOFFTRÄGER AUS NICHTIONISCHEN TENSIDEN UND IHRE VERWENDUNG,SPEZIELL IN NAHRUNGSMITTELN,KOSMETIKA UND ARZNEIMITTELN
ACTIVE PRINCIPLE CARRIERS CONTAINING NON-IONIC SURFACTANTS, AND USES THEREOF, PARTICULARLY IN FOOD, COSMETICS AND PHARMACEUTICALS

(30) Priorité: 21.06.1995 FR 9507398
(43) Date de publication de la demande: 08.04.1998
(73) Titulaire: CAPSULIS, 33600 Pessac (FR)
(72) Inventeur: ROUX, Didier, F-33700 Merignac (FR); DEGERT, Corinne, F-33160 Saint-Médard-en-Jalles (FR); LAVERSANNE, René, F-33600 Pessac (FR)
(74) Mandataire: Giraud, Françoise
(86) Numéro de dépôt international: FR9600958
(87) Numéro de publication internationale: WO9700623

(56) Documents cités:
- EP-A- 0 087 993
- EP-A- 0 489 207
- WO-A-93/19735
- DE-A- 4 421 686

## Description

L'invention concerne des véhicules de principes actifs à base de tensioactifs non ioniques et leurs applications notamment dans les domaines alimentaire, cosmétique et pharmaceutique.

Elle concerne également des compositions dans lesquelles au moins un principe actif se trouve encapsulé au sein de ce véhicule, en particulier des compositions à usage alimentaire, cosmétique ou pharmaceutique et leur procédé de fabrication.

On sait qu'il existe une nécessité importante dans ces domaines, de protéger un certain nombre de molécules fragiles ou volatiles ou de régler leurs conditions de relargage vers un milieu extérieur.

Une des techniques permettant d'atteindre un tel but est la microencapsulation de molécules actives. Cette encapsulation a pour objectif de diminuer l'évaporation et le transfert vers l'environnement de la matière active, soit au cours du stockage, soit au cours de l'élaboration des produits ou encore au cours de leur consommation.

Elle peut également permettre de rendre le matériau plus facile d'utilisation en le diluant et en favorisant sa répartition homogène au sein du support.

La liste des matériaux sensibles est longue.

Parmi les plus utilisés, on citera, par exemple dans le domaine alimentaire, les acides, par exemple l'acide ascorbique ou lactique, les colorants en particulier pour les viandes, les lipides, les vitamines, les arômes et les huiles essentielles, les enzymes.

Dans le domaine cosmétique ou pharmaceutique, on peut citer la dihydroxyacétone, les vitamines, les oligomères phénoliques, les biomolécules. Certains des actifs cités en alimentaire sont aussi utilisés en cosmétique ou en pharmacie.

Les technologies, actuellement mises en oeuvre afin d'atteindre cet objectif, reposent principalement sur l'emploi de polymères. Ce sont des techniques soit de coacervation, soit d'extrusion ou d'enrobage par lit fluidisé.

Indépendamment de l'effet dispersant, les molécules tensioactives permettent, dans certains cas, de protéger et de vectoriser puis de relarguer de façon contrôlée des molécules actives, ceci, en utilisant des microcapsules formées par une association supramoléculaire des molécules tensioactives. L'exemple le plus courant est celui des liposomes utilisés en cosmétique et dans le domaine biomédical. Ces liposomes correspondent à un arrangement en vésicules, unilamellaires ou multilamellaires, de tailles comprises entre quelques centaines d'Angströms et plusieurs microns. Ces vésicules sont, dans le cas des liposomes, obtenus à partir de molécules phospholipidiques (extraites par exemple du soja ou de l'oeuf). Ces liposomes sont capables d'encapsuler des molécules actives hydrophiles ou même lipophiles et ainsi de réaliser les fonctions de vectorisation et de relargage visées.

Les méthodes classiques de préparation des liposomes nécessitent, le plus souvent, la présence d'un cosolvant organique ou d'alcools, produits fortement déconseillés pour les applications dans le domaine alimentaire et à éviter dans les autres domaines plus particulièrement visés par la présente invention.

Toutefois, dans le domaine de l'industrie alimentaire, des essais effectués avec des lécithines, en particulier sous forme de liposomes, ont montré les limitations technologiques de cette technique. En effet, l'utilisation des liposomes dans l'industrie alimentaire est limitée par le procédé de fabrication qui présente un certain nombre de problèmes techniques. Plus précisément, l'utilisation des liposomes dans l'industrie alimentaire présente l'inconvénient de conduire à un rendement d'encapsulation faible et, ceci malgré une technologie relativement lourde et donc chère à mettre en oeuvre.

Le brevet US 4,217,344 décrit un procédé permettant de produire une dispersion de sphères comprenant un arrangement de couches moléculaires encapsulant une phase aqueuse. Le procédé décrit dans ce document consiste à mélanger un composé lipidique dispersable dans l'eau avec la phase aqueuse à encapsuler, le rapport lipophile/hydrophile du composé lipidique étant tel que le lipide gonfle dans la phase aqueuse pour former une phase lamellaire qui est ensuite soumise à une étape d'agitation.

Parmi les composés lipidiques cités dans ce document, on trouve essentiellement des composés de type éthers de polyglycérol synthétisés spécifiquement pour former des vésicules.

On a maintenant trouvé une nouvelle famille de tensioactifs de type esters permettant d'obtenir des vésicules stables permettant d'encapsuler des principes actifs.

Un avantage tout particulier des tensioactifs sélectionnés selon l'invention est qu'il s'agit de produits d'origine naturelle, biodégradables, non éthoxylés, facilement accessibles sur le marché permettant ainsi d'obtenir des microvésicules multilamellaires à partir de tensioactifs commerciaux, sans avoir à effectuer une synthèse particulière.

En effet, les liposomes, ou autres vésicules lipidiques décrites dans la littérature sont jusqu'à présent basés essentiellement sur des tensioactifs :
- ioniques ou zwitterioniques, comme c'est le cas de la lécithine et de ses dérivés, du dodécylsulfate de sodium (SDS)
- non ioniques éthoxylés, comme c'est le cas des microcapsules à base d'ester de sorbitan
- non ioniques spécialement synthétisés pour donner des vésicules.

L'utilisation de ces produits présente certains inconvénients, entre autres dans les domaines cosmétique et alimentaire :
- irritation dans le cas des tensioactifs ioniques et de certains non ioniques éthoxylés,
- risque d'allergie dû aux traces d'oxyde d'éthylène des produits éthoxylés,
- grande sensibilité à la contamination microbienne en ce qui concerne la lécithine, les produits éthoxylés qui inhibent les conservateurs,
- instabilité chimique en ce qui concerne la lécithine,
- spécificité de formulation.

Les tensioactifs, non ioniques, utilisés selon l'invention sont pour l'essentiel constitués d'esters d'acides gras et de saccharose, éventuellement en mélange avec des esters de glycérol. Il s'agit avantageusement de dérivés de produits naturels, de préférence d'origine végétale permettant d'éviter les inconvénients des produits cités précédemment. Ils n'ont pas été utilisés jusqu'à présent pour la préparation de vésicules lipidiques qui sont basées principalement sur la lécithine, des tensioactifs non ioniques éthoxylés ou des tensioactifs non ioniques nécessitant une synthèse spécifique.

Ces tensioactifs ont jusqu'à présent surtout été développés pour l'industrie alimentaire car ils sont très bien tolérés, ne présentent pas de risques d'allergie ni de transmissions de maladies d'origine animale. D'autre part, ils donnent en général une structure très douce et un toucher agréable aux préparations cosmétiques dans lesquelles ils sont introduits. De plus, grâce à leur bonne compatibilité avec la peau, ils favorisent la pénétration des actifs cosmétiques.

Ces tensioactifs se sont avérés particulièrement intéressants pour la préparation de vésicules multilamellaires, utilisées comme vecteurs d'actifs, aussi bien pour l'industrie alimentaire, que cosmétique ou pharmaceutique. Ils présentent une variété de propriétés physico-chimiques importante, qui permet de choisir parmi eux des couples de propriétés différentes (haute et basse HLB) afin de formuler correctement la phase cristal-liquide lamellaire nécessaire à l'obtention de microvésicules, comme cela ressort de la description qui suit.

Ils sont aussi connus pour diminuer les effets cytotoxiques, et donc irritants ou allergisants, d'autres tensioactifs, en particulier ioniques.

Enfin, ils sont moins sensibles à la contamination bactérienne, et présentent même pour certains, en particulier en ce qui concerne les sucroesters, des propriétés bactériostatiques. De ce fait, ces tensioactifs peuvent non seulement être utilisés en mélange entre eux pour former des microvésicules multilamellaires de tensioactifs, mais ils peuvent aussi être utilisés en complément d'autres tensioactifs pour obtenir les propriétés nécessaires à la formulation, tout en diminuant les inconvénients de ces autres tensioactifs (irritation des tensioactifs ioniques, contamination des lécithines...).

Les vésicules selon l'invention peuvent être obtenues de façon particulièrement simple par formation d'une phase cristal-liquide lamellaire et en provoquant le réarrangement des bicouches formées pour former des microcapsules. Un procédé de ce type permettant de faire des microcapsules de taille contrôlée est décrit dans la demande internationale WO 93/19735 qui décrit un procédé qui permet, grâce au recours à une étape de cisaillement d'une phase cristal-liquide lamellaire, de préparer des microcapsules de taille contrôlée, non seulement à partir de tensioactifs lipidiques susceptibles de former des liposomes mais aussi à partir des différents tensioactifs anioniques ou non ioniques et propose l'encapsulation de substances notamment biologiques dans ces capsules.

La demande internationale WO 95/19707 décrit, quant à elle, un procédé destiné à améliorer la rémanence d'une odeur et consistant à encapsuler un principe actif odoriférant au sein d'une microcapsule constituée d'un arrangement multilamellaire de bicouches concentriques séparées par un milieu aqueux. Ces microcapsules sont obtenues en préparant une phase cristal-liquide ou une suspension de phase cristal-liquide à partir d'au moins un ajout tensioactif et en provoquant le réarrangement des bicouches sous forme de microcapsules. Ce réarrangement peut être, en particulier, obtenu en utilisant le procédé décrit dans la demande internationale WO 93/19735.

Les vésicules selon l'invention peuvent être obtenues par un procédé dérivé directement de ceux décrits dans les demandes internationales WO 95/19707 ou WO 93/19735.

Selon l'invention, le produit actif que l'on souhaite protéger ou dont on veut contrôler le relargage à partir d'une composition est incorporé en quasi totalité à l'intérieur de vésicules multilamellaires que l'on désignera dans le présent mémoire indifféremment par microcapsules, microvésicules ou vésicules. Ces microcapsules sont avantageusement de forme sensiblement sphérique et sont constituées de lamelles concentriques leur conférant une structure de type "oignon".

La substance active se trouve ainsi incluse au sein même de la microcapsule, généralement dans ses membranes, le cas échéant si elle est purement hydrophile, dans l'eau interstitielle incluse à l'intérieur de la microcapsule. Mais elle fait toujours partie intégrante de la microcapsule.

Une telle encapsulation permet d'assurer les fonctions de dispersabilité et/ou de vectorisation et de relargage contrôlé du produit encapsulé.

Même si, d'une façon générale, on utilise des milieux eau/tensioactif(s) pour réaliser les microcapsules de l'invention, il n'est nullement exclu de remplacer l'eau par un solvant polaire, par exemple le glycérol.

Selon un premier avantage, le procédé de l'invention permet d'utiliser, pour réaliser l'encapsulation, une variété de tensioactifs parfaitement compatibles avec les usages visés en particulier avec les utilisations dans les domaines alimentaire, cosmétique ou pharmaceutique, notamment dermatologique.

Selon un autre avantage, la technologie proposée selon l'invention permet la préparation de vésicules ayant un très fort rendement d'encapsulation, notamment un rendement supérieur à 90 % et généralement très proche de 100 %. D'un emploi facile, cette technologie permet également la préparation de grandes quantités de produits encapsulés. De plus, elle n'utilise pas le passage par un cosolvant organique, ce qui permet d'envisager l'utilisation industrielle des vésicules multilamellaires pour microencapsuler des molécules ou des compositions à usage spécifique.

Un autre avantage vient du fait que l'utilisation des tensioactifs confère une bonne dispersabilité à la formulation qui peut être utilisée sous forme liquide, en dispersion aqueuse. Cet aspect est particulièrement avantageux lorsque l'on a affaire à des molécules hydrophobes ou non solubles dans l'eau qui peuvent être dispersées grâce à l'invention sans avoir recours à un solvant organique.

Par ailleurs, les techniques classiques de microencapsulation, en particulier des arômes, consistent à entourer un produit lipophile par une coquille polymérisée. Ceci conduit à des arômes encapsulés qui se libèrent d'un seul coup au moment de la rupture de la coque. De même, les techniques de microencapsulation moléculaire, par exemple par la cyclodextrine, conduisent à un complexe permanent et à un véritable équilibre chimique entre la forme complexée et la forme libre. Au contraire, la technologie de microencapsulation par tensioactifs selon l'invention permet un relargage sensiblement différent. En effet, on peut considérer que le produit actif, par exemple l'arôme, fuit constamment mais que la libération du produit actif est considérablement ralentie par rapport au même produit libre.

Par ailleurs, du fait de leur petite taille, de l'ordre du micromètre, ces capsules ne sont pas généralement détruites lors du processus de mastication, ce qui permet, dans le cas des produits alimentaires, une rémanence particulièrement prononcée de la sensation liée au goût du produit pendant toute la période de sa mastication.

De plus, cette technologie permet dans certains cas d'augmenter la disponibilité d'un arôme lipophile. En effet, si un arôme lipophile est mélangé dans un milieu, lui-même, lipophile, sa disponibilité dans la bouche (milieu aqueux) peut être très limitée. L'arôme est dans ce cas piégé dans son milieu et ne se libère pas. C'est le cas, par exemple, des arômes dispersés dans un produit laitier ou dans des parties grasses d'une viande ou encore dans la matrice polymère d'un chewing-gum.

Selon l'un de ses aspects, la présente invention fournit un procédé permettant de protéger, avant leur introduction dans des compositions à usage spécifique, en particulier alimentaire, cosmétique ou pharmaceutique, notamment dermatologique, des produits ou additifs fragiles et/ou dont on souhaite contrôler le relargage, en particulier pendant leur consommation. Ce procédé consiste à encapsuler ces produits ou additifs à l'intérieur de vésicules multilamellaires constituées des tensioactifs spécifiques utilisés selon l'invention.

Selon un autre aspect, l'invention concerne un véhicule de substances actives constitué de vésicules à base de tensioactifs non ioniques de type esters bien déterminés.

Selon un autre aspect, l'invention concerne des compositions à utilisation spécifique dans lesquelles la substance active se trouve incluse dans les vésicules de l'invention.

Plus précisément, l'invention concerne des compositions à usage alimentaire ou diététique dans lesquelles un produit ou un additif à usage alimentaire ou diététique se trouve microencapsulé.

L'invention concerne aussi des compositions pharmaceutiques dans lesquelles au moins une substance active se trouve incluse dans les vésicules fabriquées selon l'invention.

L'invention concerne également des compositions cosmétiques dans lesquelles au moins un actif cosmétiquement efficace est inclus dans des vésicules selon l'invention.

Elle concerne également, selon un autre aspect, des aliments incorporant un produit ou additif à usage alimentaire encapsulé.

Elle concerne également et, cela, dans la mesure où l'utilisation des tensioactifs de la famille des sucroesters et des sucroglycérides se trouve décrite pour la première fois, pour réaliser des microcapsules constituées d'un arrangement multilamellaire de bicouches concentriques, l'utilisation de ces tensioactifs particuliers pour réaliser de telles microcapsules et cela, indépendamment de la nature de l'actif encapsulé et de l'utilisation visée.

De telles compositions contenant des microcapsules à base de sucro-glycérides et/ou de sucroesters pourront tout particulièrement être utilisées dans le domaine de la cosmétologie. En effet, les tensioactifs cités ci-dessus ont l'avantage d'être particulièrement peu irritants, et de donner une texture très douce aux produits dans lesquels ils sont utilisés. Utilisés dans les microcapsules, ils donnent accès à des vecteurs d'actifs performants et particulièrement bien tolérés, utilisables aussi bien en dermocosmétique qu'en diététique.

Ainsi, selon ses caractéristiques essentielles, l'invention concerne un véhicule de principe actif sous forme de vésicules multilamellaires constituées de membranes concentriques, caractérisé en ce que lesdites membranes comprennent au moins un tensioactif non-ionique de type ester de saccharose comprenant au moins une chaîne provenant d'un acide gras en C₁₂ à C₂₂, linéaire ou ramifié, saturé ou insaturé, éventuellement mono- ou polyhydroxylé.

Les acides gras entrant dans la composition des tensioactifs utilisés pour préparer les vésicules selon l'invention peuvent être tout acide gras en C₁₂ à C₂₂ cité ci-dessus. Toutefois, on choisira avantageusement les acides gras d'origine naturelle, de préférence végétale, plus précisément ceux trouvés dans les huiles végétales. Il peut s'agir soit d'acide gras défini, soit de mélanges d'acides gras, en particulier de mélanges issus d'huile naturelle, en particulier d'huile d'olive, d'arachide, de colza, de ricin, de palme, de coprah, de sésame. Ces acides gras peuvent être partiellement ou totalement hydrogénés. On pourra, en particulier, utiliser différents acides gras trouvés en mélange en proportions variables dans les huiles citées ci-dessus et constitués, pour l'essentiel, d'acides gras en C₁₂ à C₂₂, saturés, mono-insaturés ou poly-insaturés.

Selon une variante de l'invention, le tensioactif de type ester utilisé selon l'invention est un ester mixte comprenant, outre les chaînes provenant des acides gras cités précédemment, au moins une chaîne provenant d'un acide carboxylique à chaîne relativement courte. Ces chaînes proviennent de mono- ou de polyacides en C₂ à C₄, saturés ou insaturés, hydroxylés ou non, la(les) fonction(s) hydroxyle(s) étant libre(s) ou estérifiée(s) par un acide carboxylique. A titre d'exemple de tels acides, on citera les acides acétique, lactique, citrique, tartrique, acétyltartrique, succinique.

Selon une autre variante de l'invention, les tensioactifs précédemment définis peuvent être utilisés en mélange avec des tensioactifs de type glycéride, ce mélange constituant, ce que l'on a coutume d'appeler sous le vocable "sucroglycérides". Les sucroglycérides utilisés selon l'invention peuvent être tout sucroglycéride susceptible d'être obtenu par transestérification partielle d'un mono- , d'un di- ou d'un triglycéride d'un acide gras en C₁₂ à C₂₂ tel que défini précédemment avec du saccharose.

Outre les tensioactifs définis précédemment, les vésicules selon l'invention pourront contenir au moins un agent tensioactif tel qu'une lécithine, un ester de sorbitan ou un polysorbate.

Le véhicule selon l'invention est, plus précisément, constitué de microcapsules constituées d'un arrangement multilamellaire de bicouches concentriques constituées d'au moins un tensioactif et séparées par un milieu constitué d'un liquide polaire dit solvant interstitiel, le produit actif se trouvant inclus dans les membranes desdites microcapsules et/ou dans le solvant interstitiel desdites microcapsules.

Selon une variante particulièrement avantageuse de l'invention, le liquide polaire est constitué par de l'eau et le produit ou additif actif est inclus dans les membranes desdites microcapsules lorsqu'il est hydrophobe et/ou dans le solvant interstitiel lorsqu'il est hydrophile.

Les microcapsules contenues dans les compositions ci-dessus ont avantageusement des dimensions comprises entre 0,1 et 50 µm, de préférence entre 0,2 et 10 µm.

Ces microcapsules peuvent être observées avec un microscope optique. Elles ont avantageusement une taille de préférence de l'ordre du micromètre. Du fait de cette faible taille, les microcapsules sont soumises à l'agitation brownienne et ne subissent pas ou très peu de décantation ou de crémage en solution aqueuse, ce qui est un avantage tout particulier de l'invention.

Comme on l'a vu précédemment, les microcapsules de l'invention ont une structure multilamellaire, c'est-à-dire une structure en oignon constituée d'une succession de couches concentriques de tensioactifs.

On choisit, de préférence, pour la préparation des microcapsules utiles selon l'invention des tensioactifs compatibles avec un usage dans le domaine visé.

Pour la mise en oeuvre du procédé de l'invention qui sera décrite plus loin, on choisit avantageusement d'utiliser, pour former les membranes des microcapsules, au moins deux tensioactifs dont l'un au moins et, de préférence, au moins deux, est un tensioactif de type sucroester tel que défini précédemment ou un mélange en contenant, en particulier un sucroglycéride. Plus précisément, lorsqu'on choisit un mélange de deux tensioactifs, on choisit de préférence un mélange comprenant un premier agent tensioactif dit agent lipophile présentant une balance hydrophile-lipophile (HLB) comprise entre 3 et 7 et un deuxième agent tensioactif dit agent hydrophile présentant une HLB comprise entre 8 et 15, l'un au moins de ces deux agents tensioactifs appartenant à la famille des sucroglycérides ou sucroesters tels que définis précédemment.

Dans ce cas, on choisira de préférence au moins un tensioactif présentant une concentration micellaire critique (CMC) inférieure à 10-5 mole/l de préférence inférieure à 10-6 mole/l.

La proportion de tensioactif lipophile dans la membrane est, exprimée en pourcentage en poids, par rapport à l'ensemble des tensioactifs, comprise entre 0 % et 100 %, de préférence entre 20 % et 100 %, le complément étant constitué du tensioactif hydrophile.

Selon une variante particulièrement avantageuse, l'un au moins des tensioactifs, et de préférence les deux, est choisi dans le groupe constitué par :
- les esters de saccharose et d'acides gras en C12 à C22, linéaires ou ramifiés, saturés ou insaturés, éventuellement mono- ou polyhydroxylés,
- des mono-, di- et triesters de glycérol des mêmes acides gras,
- les esters mixtes de glycérol des mêmes acides gras et de mono- ou polyacides en C2 à C4 saturés ou insaturés, hydroxylés ou non, le(s) fonction(s) hydroxyle(s) étant libre(s) ou estérifié(s) par un acide carboxylique, par exemple l'acide acétique, lactique, citrique, tartrique, acétyltartrique, succinique,
- les sucroglycérides formés d'un mélange d'esters de saccharose et de mono- ou diglycérides préparés avec les mêmes acides gras.

Comme tensioactif hydrophile, on choisira tout tensioactif hydrophile tel que défini précédemment.

La famille des tensioactifs de type sucroester sera particulièrement préférée.

On choisira de préférence des sucroesters d'acides gras issus de matières grasses végétales.

D'une façon générale, les tensioactifs selon l'invention contiendront avantageusement des esters simples ou mixtes ou des mélanges d'esters d'acide gras choisis indépendamment de préférence parmi l'acide laurique, l'acide myristique, l'acide palmitique, l'acide stéarique, l'acide béhénique, l'acide oléique, l'acide linoléique, l'acide linolénique, l'acide arachidonique, l'acide ricinoléique et leurs mélanges.

Les vésicules décrites précédemment peuvent être renforcées par enrobage au moyen d'un polymère, avantageusement un polymère d'origine naturelle, végétale ou marine, ou coencapsulation d'un tel polymère avec l'actif. Un tel enrobage ou une telle coencapsulation permet de conserver les caractères spécifiques des vésicules selon l'invention à savoir les caractères de tolérance, de douceur et de compatibilité des tensioactifs décrits.

Les polymères avantageusement utilisés pour enrober les vésicules ou pour être coencapsulés avec un actif de façon à renforcer lesdites vésicules sont avantageusement choisis dans les classes suivantes de polymères :
- les polysaccharides d'origine naturelle ou modifiée, linéaires ou substitués, neutres ou ioniques, en particulier les gommes guar, les gommes de caroube, la gomme arabique, les carraghénanes (kappa, iota et lambda), les gommes de xanthane, les pectines, natives, estérifiées ou amidées, l'acide alginique et ses sels, l'acide hyaluronique, les gommes guar quaternisées, le chitosan et ses dérivés substitués,
- la gélatine,
- les polymères synthétiques hydro- ou liposolubles tels que le polyacrylamide, la polyvinylpyrrolidone, le polyéthylèneglycol.

Comme on l'a exposé précédemment, l'invention concerne également différentes compositions utilisant les vésicules précédemment décrites comme véhicule d'actif. Il s'agit, en particulier, de compositions à usage alimentaire ou diététique, de compositions pharmaceutiques ou de compositions cosmétiques.

Les produits ou additifs à usage alimentaire ou diététique, que l'on encapsule selon la présente invention sont tous les produits connus comme additifs à usage alimentaire, connus pour leur fragilité. Il peut s'agir aussi bien de produits à caractère hydrophile qu'hydrophobe.

A titre d'exemples de produits à usage alimentaire que l'on encapsulera selon la présente invention, on peut citer en particulier des produits de type acide à usage alimentaire, notamment l'acide ascorbique ou l'acide lactique, les colorants, en particulier les colorants pour viande, les lipides, les vitamines, les arômes alimentaires, les huiles essentielles, des enzymes.

Lorsque l'actif alimentaire a un caractère hydrophile, sa concentration dans la capsule, exprimée en pourcentage en poids par rapport au poids total de la capsule incluant les poids du liquide polaire, du (des) tensioactif(s) et du produit actif encapsulé, est généralement comprise entre 5 et 30 %, de préférence entre 10 et 20 %. Lorsque le produit actif est hydrophobe, sa concentration dans la capsule est généralement comprise entre 5 et 30 %, de préférence entre 5 et 20 %.

L'invention concerne également des compositions cosmétiques dans lesquelles un agent actif, en particulier un agent actif dont on souhaite améliorer la pénétration à travers la peau, se trouve inclus à l'intérieur des vésicules décrites précédemment.

L'invention concerne également des compositions pharmaceutiques contenant au moins un agent actif inclus à l'intérieur des vésicules telles que décrites précédemment, en particulier un agent actif dont on souhaite améliorer ou contrôler le relargage ou, plus particulièrement dans le cas des compositions à usage dermatologique, un agent actif dont on souhaite contrôler la pénétration à travers la peau.

A titre d'exemple, on peut citer les actifs hydratants, ou anti-radicaux libres pour la cosmétique, les anti-inflammatoires, les anesthésiques locaux, les anti-allergiques, les antalgiques pour la pharmacie.

Dans les compositions cosmétiques ou pharmaceutiques, les concentrations en actif peuvent considérablement varier en fonction de la nature de cet actif. D'une façon générale, elles sont comprises entre 5 et 60 % par rapport au poids total de la capsule. Des pourcentages relativement élevés, par exemple de 50 à 60 % pouvant, en particulier, être utilisés lorsque l'actif remplace l'eau intersticielle ; c'est par exemple le cas du glycérol utilisé comme agent hydratant en cosmétique.

Selon un autre de ses aspects, l'invention concerne également un procédé de préparation des compositions décrites précédemment. Ce procédé consiste à préparer une phase lamellaire cristal-liquide contenant au moins un agent tensioactif, de type sucroester tel que défini précédemment, un solvant polaire, avantageusement constitué par de l'eau, et le produit ou la composition que l'on souhaite encapsuler et à provoquer le réarrangement de ladite phase cristal-liquide sous forme de vésicules multilamellaires.

Plus précisément, la technique de préparation consiste, dans une première étape, à préparer une phase lamellaire cristal-liquide contenant un mélange du tensioactif ou des tensioactifs, du solvant polaire, de l'eau de préférence, et du produit ou mélange actif que l'on cherche à encapsuler.

Pour optimiser le rendement d'encapsulation, on choisira des conditions telles que la phase cristal-liquide soit homogène, c'est-à-dire monophasique, de façon à ce que l'ensemble du solvant polaire (eau en général), du produit ou mélange actif soit solubilisé dans cette phase lamellaire.

Les conditions optimales à utiliser pourront être en général déterminées par examen d'une série de compositions contenant des quantités variables de solvant et de produit actif. Cet examen sera fait soit par observation macroscopique en observant la séparation de phase, soit par observation microscopique en utilisant un microscope optique, de préférence un microscope polarisant.

Toutefois, la formation d'une phase cristal-liquide n'est pas une condition suffisante pour obtenir, par la suite, une suspension aisément dispersable de vésicules multilamellaires. Il faut, de plus, obtenir une organisation de cette phase lamellaire sous la forme d'un empilement compact de ces vésicules. Ce réarrangement pourra être obtenu en appliquant un cisaillement homogène, comme décrit dans la demande de brevet WO 93/19735. Ce réarrangement pourra être également obtenu en jouant sur la formulation particulière du mélange, en particulier en choisissant un mélange de tensioactifs tels que la texture recherchée, sous forme de vésicules multilamellaires, se forme spontanément ou à défaut lors d'une simple sollicitation mécanique, par exemple lors du mélange des produits qui provoque une telle sollicitation mécanique.

Cest pourquoi, on choisira avantageusement un mélange de tensioactifs et des concentrations respectives de chacun des tensioactifs contenus dans ce mélange de façon à obtenir la texture désirée.

Plus précisément, on utilisera un mélange de tensioactifs constitué généralement de deux types de tensioactifs, l'un étant plutôt soluble dans l'eau et présentant donc une HLB élevée et l'autre étant plutôt soluble dans l'huile et présentant donc une HLB relativement basse. Par ailleurs, il sera particulièrement avantageux que l'un au moins des tensioactifs ait une CMC relativement basse, de préférence inférieure à 10⁻⁵ mole/litre, de préférence encore inférieure à 10⁻⁶ mole/l.

La proportion en poids des tensioactifs dans le mélange final se situe généralement entre 5 et 90 %, de préférence entre 30 et 70 %.

Plus précisément, pour obtenir les microcapsules recherchées, on utilisera des mélanges de départ présentant les propriétés suivantes :
1) Le mélange doit former une phase lamellaire cristal-liquide homogène pour des proportions d'eau, en poids, allant de 10 à 98 %, plus généralement de 20 à 60 %.
2) Cette phase lamellaire homogène doit présenter une texture spécifique, c'est-à-dire un arrangement spatial des lamelles qui, soit spontanément, soit sous simple mélange, soit encore sous l'action d'un cisaillement spécifique tel que décrit dans la demande internationale WO 93/19735, corresponde à une structure en "oignon". Cette structure peut être reconnue aisément par l'homme de métier en utilisant un microscope polarisant.

Pour obtenir les deux conditions ci-dessus, on utilisera avantageusement, comme on l'a expliqué précédemment, deux tensioactifs ayant des équilibres hydrophile/lipophile sensiblement différents, afin de pouvoir ainsi régler à loisir les propriétés d'organisation (texture) de la phase lamellaire.

On choisira de préférence de mélanger un tensioactif plutôt lipophile présentant une HLB basse comprise entre 3 et 7 et un tensioactif hydrophile présentant une HLB élevée comprise entre 8 et 15. L'homme du métier pourra aisément, en faisant varier les proportions des deux types de tensioactifs, obtenir une phase lamellaire homogène ayant des propriétés de texture recherchées.

Les deux types de tensioactifs seront choisis parmi les tensioactifs compatibles avec l'usage visé.

Ainsi, on choisira avantageusement le tensioactif de type lipophile dans la famille comprenant les mono-, di- et triglycérides ou leurs dérivés, en particulier leurs dérivés de type ester et les sucroglycérides.

Le tensioactif de type hydrophile sera, de façon particulièrement avantageuse, choisi dans la famille des sucroesters.

Dans certains cas particuliers, on peut obtenir ces capsules à partir d'un seul produit commercial. C'est le cas du produit commercialisé, par la société Rhône-Poulenc sous le nom de celynol PPH, qui est un sucroglycéride hydrogéné.

En appliquant le procédé de préparation précédemment décrit, on aboutit à deux types de capsules selon le degré d'organisation des molécules tensioactives dans la membrane constituant les compartiments des vésicules multilamellaires :
- les vésicules de type "fluide" correspondent à des membranes où les molécules de tensioactifs sont libres de se mouvoir et ne sont pas organisées sous forme d'un réseau cristallin bidimensionnel. Elles ont, en général, une forme sphérique.
- les vésicules de type "solide" correspondent, au contraire à une organisation des molécules de tensioactif sous la forme d'un réseau cristallin bidimensionnel. La forme de ces vésicules est anisotrope et se présente le plus souvent sous la forme de petits cristaux facettés. Dans tous les cas, la taille des vésicules est comprise en 0.1 et 50 µm. L'aspect facetté de ces vésicules n'est pas contradictoire avec leur structure multilamellaire de type oignon.

Selon une variante de l'invention, on peut, comme exposé précédemment, enrober les microcapsules décrites précédemment d'un polymère réticulé, ce qui permet d'améliorer les qualités des capsules formées.

On peut également encapsuler par la méthode précédemment décrite un agent réticulant d'un polymère qui s'adsorbe sur les capsules. On peut réaliser cette opération en incorporant un sel de calcium agissant comme agent réticulant dans l'eau permettant de former les capsules. En diluant ces capsules dans une solution d'alginate, on peut ainsi former des capsules multilamellaires recouvertes du polymère (alginate) réticulé par le calcium. Ceci permet de limiter la fuite d'un produit hydrophile et de stabiliser les microcapsules.

Une autre méthode consiste à co-encapsuler par la méthode précédemment décrite un polymère capable d'être réticulé par un agent chimique, par exemple un alginate. La dispersion des capsules dans une solution de l'agent réticulant (par exemple dans le cas de l'alginate, une solution de sel de calcium) conduit, par la diffusion du réticulant à l'intérieur des capsules à la réticulation du polymère encapsulé. On obtient ainsi des capsules solidifiées par l'incorporation d'un polymère réticulé, ce qui diminue considérablement la fuite du principe actif.

D'autres polymères naturels peuvent être utilisés à cette fin. On peut citer la gomme guar, dont l'agent de réticulation est le borax, la pectine, réticulée par les ions Ca⁺⁺, ou les carrhagénanes, réticulés suivant leur type par les ions potassium ou les ions calcium.

### EXEMPLES

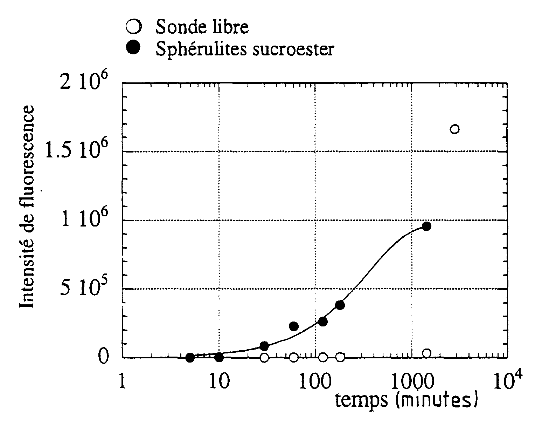

Les exemples qui suivent illustrent la présente invention. L'exemple 19 est donné en référence à la figure unique qui représente, en échelle semi-log, les variations de l'intensité de fluorescence d'une sonde ayant traversé une barrière cutanée (épiderme humain reconstruit), sous forme libre ou sous forme encapsulée.

### Exemple 1 : Préparation de microcapsules lécithine/sucroesters

On mélange 10 g de monopalmitate de saccharose commercialisé par la société Ryoto sous la marque P1570 ou P1670 à 50 g d'eau dans un erlenmeyer à 60°C. On attend la dissolution complète puis on ajoute 40 g de lécithine de soja commercialisée sous la marque Mactan P97 par la société Norte que l'on mélange à chaud. On laisse la température revenir à l'ambiante. On obtient une pâte homogène qui peut être facilement dispersée soit dans l'eau par simple agitation, soit dans un milieu hydrophobe tel que de l'huile. On peut observer sous microscope la présence de particules sphériques qui correspondent aux vésicules multilamellaires. La dispersion dans l'eau reste stable et homogène dans le temps. Au contraire, la dispersion dans l'huile se déstabilise par floculation des vésicules qui sédimentent mais peuvent être simplement redispersées par sollicitation mécanique.

### Exemple 2 : Préparation de microcapsules sucroesters/monoglycérides

On introduit dans un erlenmeyer 10 g d'un mélange de mono et de diglycérides commercialisé par la société Witco sous la marque Atmos 300 et 40 g de monopalmitate de saccharose commercialisé par la société Ryoto sous la marque P1570 ou P1670 et 50 g d'eau. On mélange à chaud (60°C), puis on laisse refroidir sous agitation à température ambiante. On obtient une pâte homogène qui peut être facilement dispersée dans de l'eau par simple agitation. On peut observer sous microscope la présence de particules sphériques qui correspondent aux vésicules multilamellaires.

### Exemple 3 : Encapsulation de colorants alimentaires

On mélange dans un erlenmeyer à 60°C 10 g de monopalmitate de saccharose commercialisé par la société Ryoto sous la marque P1570 ou P1670 à 50 g d'eau dans laquelle on a préalablement dispersé 1 g de colorant alimentaire (E124). On attend l'incorporation complète, puis on ajoute 40 g de lécithine que l'on mélange à chaud. On laisse la température revenir à l'ambiante. On obtient une pâte homogène colorée qui peut être facilement dispersée dans l'eau par simple agitation. On peut observer sous microscope la présence de particules sphériques qui correspondent aux vésicules multilamellaires. On peut par centrifugation séparer une partie des capsules. On vérifie ainsi en récupérant des capsules fortement colorées que le colorant, bien qu'hydrophile, reste piégé dans les capsules.

### Exemple 4 : Encapsulation d'arôme de menthe

On introduit dans un erlenmeyer 10 g d'un mélange de mono- et de diglycéride commercialisé sous la marque Atmos 300 par la société Witco, 90 g de monopalmitate de saccharose commercialisé par la société Ryoto sous la marque P1570 ou P1670, 40 g d'arôme de menthe et 60 g d'eau. On mélange à chaud (60°C) puis on laisse refroidir sous agitation à température ambiante. On obtient une pâte homogène qui peut être facilement dispersée dans de l'eau par simple agitation. On peut utiliser cette pâte directement dans des préparations. Il est possible d'observer un effet de rémanence de l'arôme par exemple en laissant s'évaporer une solution aqueuse de cet arôme et en comparant l'arôme encapsulé à une solution d'arôme non encapsulé.

### Exemple 5 : Encapsulation d'arôme de fruits

On mélange dans un erlenmeyer à 60°C 10 g de monopalmitate de saccharose commercialisé par la société Ryoto sous la marque P1570 ou P1670 à 30 g d'eau. On attend la dissolution complète, puis on ajoute 40 g de lécithine et 20 g d'arôme de fruits que l'on mélange à 40° C. On laisse la température revenir à l'ambiante. On obtient une pâte homogène colorée qui peut être facilement dispersée dans l'eau par simple agitation. On peut observer sous microscope la présence de particules sphériques qui correspondent aux vésicules multilamellaires.

### Exemple 6 : Capsules d'arôme de fruit/sucroglycérides hydrogénés

On met dans un bécher 50 g de sucroglycéride hydrogéné commercialisé par la société Rhône-Poulenc sous la marque Celynol PPH, 38 g d'eau et 12 g d'arôme de fruits. On chauffe en mélangeant à 60° C et on agite pendant 20 mn. Puis on refroidit. On obtient une pâte de capsules concentrées contenant l'arôme. Ces capsules sont dispersables dans l'eau par une agitation forte.

### Exemple 7 : Capsules d'arôme de fruit/sucroglycéride/sucroester

On met dans un bécher 25 g de sucroglycéride commercialisé par la société Rhône-Poulenc sous la marque Celynol LMO, 25 g de P1670 (de chez Ryoto), 38 g d'eau. On chauffe en mélangeant à 60° C et on agite pendant 20 mn. On refroidit. Lorsque la température atteint 40° C, on peut ajouter 12 g d'arôme. On agite 30 mn, puis on refroidit. On obtient une pâte de capsules concentrées contenant l'arôme. Ces capsules sont dispersables dans l'eau par une agitation forte.

### Exemple 8 : Capsules de stéaroyl-2-lactilate de sodium/sucroglycérides contenant un arôme de fruit

On met dans un bécher 30 g de stéaroyl-2-lactylate de sodium commercialisé par la société Witco sous la marque Emulsilac S, 20 g de sucroglycéride hydrogéné commercialisé par la société Rhône-Poulenc sous la marque Celynol PPH, 38 g d'eau et 12 g d'arôme de fruits. On chauffe en mélangeant à 60° C et on agite pendant 20 mn. Puis on refroidit. On obtient une pâte de capsules concentrées contenant l'arôme. Ces capsules sont dispersables dans l'eau par une agitation forte.

### Exemple 9 : Microcapsules formées à partir d'un solvant non aqueux

On met dans un bécher 40 g de lecithine, 10 g de monopalmitate de saccharose commercialisé sous la marque P1670 par la société Ryoto, 50 g de glycérol. On chauffe à 60° C en agitant pendant 30 mn. On refroidit. On obtient une pâte de capsules sans eau.

### Exemple 10 : Microcapsules contenant un aromate utilisé en charcuterie

On prépare des microcapsules similaires à celles de l'exemple 1, à partir de 15 g de monopalmitate de saccharose P1670, de 35 g de lécithine de soja, de 45 g d'eau et de 5 g d'arôme artificiel de saucisson obtenu à partir de 1-octen-3-ol. La pâte obtenue est dispersée dans l'eau pour faire des dispersions contenant 1 %, 0,1 % et 0,01 % d'arôme. Ces dispersions sont utilisées à raison de 10 g de dispersion par kg de viande pour la fabrication de saucissons. Une analyse sensorielle montre que les saucissons fabriqués en incorporant la dispersion à 0,1 % d'arôme ont un goût développé de salaison, alors qu'un témoin sans arôme n'a pas encore développé ce goût.

### Exemple 11 : Microcapsule à base d'ester de saccharose et de monoglycéride

Dans un erlen de 100 cm³ on introduit 5 g de monoglycéride commercial d'huile de tournesol et 7,5 g d'acétate de vitamine E. Le mélange est homogénéisé sous agitation mécanique. Lorsque la pâte est homogène, on ajoute lentement sous agitation 20 g de palmitate commercial de saccharose, puis 17,5 g d'eau. Le produit est homogénéisé sous forte agitation à température ambiante pendant au moins 30 min, puis chauffé à 65°C en 1 à 2 h. Après refroidissement sous agitation, on obtient une phase concentrée formée de microvésicules selon l'invention que l'on peut disperser sous agitation par addition lente du même volume d'eau. L'observation au microscope optique montre une population dense de microvésicules de taille variant de 0,5 à quelques micromètres.

### Exemple 12 : Microcapsules contenant un polymère réticulé

Une solution à 0,5 % d'alginate de sodium est préparée en dispersant le polymère en poudre sous forte agitation dans l'eau à température ambiante, puis en laissant sous agitation pendant 1 h. Dans un erlen de 100 cm³ on introduit 5 g de monoglycéride d'huile de tournesol (Dimodan LS, Grindsted) et 7,5 g d'acétate de vitamine E. Le mélange est homogénéisé sous agitation mécanique. Lorsque la pâte est homogène, on ajoute lentement sous agitation 20 g de palmitate de saccharose puis 17,5 g de la solution d'alginate de sodium précédemment préparée. Le produit est homogénéisé sous forte agitation à température ambiante pendant au moins 30 min, puis chauffé à 65°C en 1 à 2 h. Après refroidissement sous agitation, on obtient une phase concentrée formée de microvésicules que l'on disperse sous agitation par addition lente du même volume d'une solution aqueuse de CaCl₂ à 0,5 %. L'observation au microscope optique de la dispersion montre les vésicules contenant le polymère réticulé, qui peuvent être séparées par centrifugation et montrent une grande rigidité.

### Exemple 13 : Microcapsules contenant un polymère réticulé

Une solution de gomme guar à 1 % est préparée en dispersant la gomme en poudre sous forte agitation dans de l'eau à température ambiante, puis en laissant sous agitation pendant 1 h. Cette solution est utilisée dans une préparation identique à celle de l'exemple 12. La pâte formée de vésicules est dispersée dans une solution aqueuse de borate de sodium à 1 %. On obtient ainsi des microvésicules encapsulant le polymère réticulé.

### Exemple 14 : Microcapsules d'ester de saccharose et de glycéride

Le mode opératoire est identique à celui de l'exemple 11, mais avec les proportions suivantes :
palmitate de saccharose : 15 g
monoglycéride d'huile de tournesol : 7,5 g
acétate de vitamine E : 5 g
extrait hydroglycolique de fleurs (bleuet, camomille, tilleul) : 22,5 g (à la place de l'eau).

La pâte obtenue en fin de préparation est dispersée dans 3 fois son volume d'eau, afin d'obtenir une dispersion fluide laiteuse de microvésicules.

### Exemple 15 : Microcapsules d'ester de saccharose et de glycéride

Le mode opératoire est identique à celui de l'exemple 11, mais avec les proportions suivantes :
palmitate de saccharose : 17,5 g
monoglycéride d'huile de tournesol : 5 g
acétate de vitamine E : 5 g
extrait aqueux d'α-hydroxyacides marins : 22,5 g.

La dispersion dans le même volume conduit à une crème contenant les microvésicules d'α-hydroxyacides, de pH voisin de 4.

### Exemple 16 : Composition pour déodorant

Dans un erlenmeyer de 100 cm³, on introduit 22,5 g de palmitate de saccharose, 5 g d'acétate de vitamine E, 3 g de 2,4,4'-trichloro-2'-hydroxybiphényléther commercial, 10 g de base parfumante pour déodorant et 9,5 g d'eau. Le mélange est homogénéisé sous forte agitation à température ambiante, puis chauffé à 65°C sous agitation pendant au moins 1 h. Après refroidissement sous agitation, on obtient une pâte contenant les microvésicules de l'invention. Cette pâte est introduite sous agitation dans une préparation pour déodorant corporel solide, sous forme de gel de stéarate aqueux, couramment désignée sous le vocable anglais "stick" On obtient ainsi un "stick" déodorant corporel contenant les microvésicules encapsulant le parfum et le bactéricide.

### Exemple 17 : Microcapsules à base d'ester de saccharose

Dans un erlenmeyer de 100 cm³, on introduit 15 g de monostéarate de saccharose et 35 g d'eau. Le mélange est homogénéisé sous forte agitation mécanique à température ambiante pendant au moins 30 min, puis est chauffé à 65°C en 1 à 2 h sous forte agitation. Après refroidissement, on obtient une crème concentrée contenant des microvésicules qui peut être dispersée aussi bien par addition lente d'eau sous agitation mécanique, que par addition lente d'une huile végétale dans les mêmes conditions. La dispersion dans l'huile présente une tendance à la sédimentation liée à la moins bonne efficacité de l'agitation brownienne dans ce milieu.

### Exemple 18 : Microcapsules à base d'esters de saccharose

Dans un erlenmeyer de 100 cm³, on introduit 20 g de stéarate de saccharose et 5 g de palmitate de saccharose. On ajoute lentement sous agitation 50 g d'eau. Le mélange est homogénéisé sous forte agitation mécanique à température ambiante pendant au moins 30 min, puis est chauffé à 65°C en 1 à 2 h. Après refroidissement, on obtient une crème concentrée contenant des microvésicules de l'invention.

### Exemple 19

Une préparation de microvésicules fluorescentes est effectuée selon le mode opératoire de l'exemple 11, à partir des matières suivantes :
palmitate de saccharose : 20 g
monoglycéride d'huile de tournesol : 5 g
acétate de vitamine E : 7,5 g
dilaurate de fluorescéine : 0,5 g
eau : 17 g.

La pâte concentrée de microvésicules est dispersée à 1 % dans l'eau. Cette dispersion est testée pour sa faculté à traverser un épiderme humain reconstruit, par rapport à la même concentration de sonde fluorescente solubilisée dans le di(éthylèneglycol)éthyléther (Transcutol).

Le test est effectué sur un modèle cellulaire (Episkin, Saduc) de peau humaine reconstruite. La solution étudiée est une solution à 10 % de la dispersion précédente, ou une dilution aqueuse à 10 % de la solution de dilaurate de fluoréscéine dans le transcutol. La concentration de sonde est donc de 10⁻⁵. L'essai est réalisé en triplica sur 48 h, et le passage de la sonde est mesuré par spectroscopie de fluorescence, après révélation de la fluorescence de la sonde en milieu basique. Les résultats sont résumés sur la courbe de la figure unique représentant l'intensité de fluorescence en fonction du temps en échelle semi-log. On remarque la très nette accélération de la cinétique de passage des microvésicules par rapport à la sonde libre. Un test de cytotoxicité est réalisé à la fin de l'expérience, suivant un protocole utilisant le bromure de diméthylthiazoldiphényltétrazolium (MTT) utilisant le kit MTT, Saduc. Il montre une légère cytotoxicité de la sonde libre à 48 h, qui a disparu dans la sonde encapsulée. Cet effet est à corréler avec l'accélération du passage de la sonde libre à 48 h, qui n'apparaît plus sur la sonde encapsulée.

## Revendications

1. Véhicule de principe actif sous forme de vésicules multilamellaires constituées de membranes concentriques, **caractérisé en ce que** lesdites membranes comprennent au moins un tensioactif non-ionique de type ester de saccharose comprenant au moins une chaîne provenant d'un acide gras en C₁₂ à C₂₂, linéaire ou ramifiée, saturée ou insaturée, éventuellement mono- ou polyhydroxylée.

2. Véhicule selon la revendication 1, **caractérisé en ce que** ledit acide gras est un acide gras d'origine naturelle, de préférence végétale.

3. Véhicule selon l'une des revendications 1 ou 2, **caractérisé en ce que** ledit ester comprend en outre au moins une chaîne carboxylique provenant d'un mono- ou polyacide en C₂ à C₄, saturé ou insaturé, hydroxylé ou non, la(les) fonction(s) hydroxyle(s) étant libre(s) ou estérifiée(s) par un acide carboxylique, par exemple l'acide acétique, lactique, citrique, tartrique, acétyltartrique, succinique.

4. Véhicule selon l'une des revendications 1 à 3, **caractérisé en ce que** ledit ester est en mélange avec au moins un mono-, di- ou triester de glycérol et d'acide gras en C₁₂ à C₂₂, linéaire ou ramifié, éventuellement mono- ou polyhydroxylé, ledit mélange étant dénommé sucroglycéride.

5. Véhicule selon l'une des revendications 1 à 4, **caractérisé en ce qu'**il contient, en outre, au moins un autre tensioactif, par exemple la lécithine, un ester de sorbitan ou un polysorbate.

6. Véhicule selon l'une des revendications 1 à 5, **caractérisé en ce que** lesdits acides gras sont choisis indépendamment dans le groupe constitué de l'acide laurique, l'acide myristique, l'acide palmitique, l'acide stéarique, l'acide béhénique, l'acide oléique, l'acide linoléique, l'acide linolénique, l'acide arachidonique, l'acide ricinoléique et leurs mélanges.

7. Véhicule selon l'une des revendications 1 à 6, **caractérisé en ce que** lesdites vésicules sont constituées d'un arrangement multilamellaire de bicouches concentriques comprenant au moins un tensioactif de type ester et séparées par un milieu constitué d'un liquide polaire dit solvant interstitiel.

8. Véhicule selon l'une des revendications 1 à 7, **caractérisé en ce que** lesdites vésicules ont des dimensions comprises entre 0,1 et 50 µm, de préférence entre 0,2 et 10 µm.

9. Véhicule selon l'une des revendications 1 à 8, **caractérisé en ce que** les membranes desdites vésicules comprennent un mélange d'au moins deux tensioactifs dont l'un au moins est un tensioactif de type ester de saccharose tel que défini dans l'une des revendications 1 à 4, lesdits tensioactifs comprenant un premier agent tensioactif dit agent lipophile présentant une balance hydrophile-lipophile (HLB) comprise entre 3 et 7 et un deuxième agent tensioactif dit agent hydrophile présentant une HLB comprise entre 8 et 15.

10. Véhicule selon la revendication 9, **caractérisé en ce que** l'un au moins des agents tensioactifs a une concentration micellaire critique (CMC) inférieure à 10-5 mol/l, de préférence inférieure à 10-6 mol/l.

11. Véhicule selon l'une des revendications 9 ou 10, **caractérisé en ce que** l'agent tensioactif dit agent lipophile est présent en proportions exprimées en pourcentages en poids par rapport à l'ensemble des tensioactifs comprises entre 0 et 100 %, de préférence entre 20 et 100 %.

12. Véhicule selon l'une des revendications 1 à 11, **caractérisé en ce que** lesdites vésicules contiennent au moins un tensioactif appartenant à la famille des sucroesters d'acides gras issus de matières grasses végétales.

13. Véhicule selon l'une des revendications 1 à 12, **caractérisé en ce que** lesdites vésicules comprennent en outre un polymère destiné à les renforcer, ledit polymère étant soit utilisé pour enrober lesdites vésicules, soit encapsulé en leur sein.

14. Véhicule selon la revendication 13, **caractérisé en ce que** ledit polymère est choisi dans les classes suivantes de polymères :
- les polysaccharides d'origine naturelle ou modifiée, linéaires ou substitués, neutres ou ioniques, en particulier les gommes guar, les gommes de caroube, la gomme arabique, les carraghénanes (kappa, iota et lambda), les gommes de xanthane, les pectines, natives, estérifiées ou amidées, l'acide alginique et ses sels, l'acide hyaluronique, les gommes guar quaternisées, le chitosan et ses dérivés substitués,
- la gélatine,
- les polymères synthétiques hydro ou liposolubles tels que le polyacrylamide, la polyvinylpyrrolidone, le polyéthylèneglycol.

15. Composition à usage alimentaire ou diététique, **caractérisée en ce qu'**elle contient au moins un produit actif encapsulé dans des vésicules constituant le véhicule selon l'une des revendications 1 à 14.

16. Composition selon la revendication 15, **caractérisée en ce que** ledit produit actif est choisi dans la famille constituée des produits de type acide à usage alimentaire, notamment l'acide ascorbique ou l'acide lactique, des colorants, en particulier les pigments pour viande, des lipides, des vitamines, des arômes alimentaires, des huiles essentielles, des enzymes.

17. Composition cosmétique ou pharmaceutique, notamment dermatologique, **caractérisée en ce qu'**elle comprend au moins un principe actif encapsulé dans des vésicules constituant le véhicule tel que défini selon l'une des revendications 1 à 14.

18. Utilisation de tensioactifs appartenant à la famille des sucroesters et des sucroglycérides pour la fabrication d'un véhicule selon l'une des revendications 1 à 14 ou d'une composition selon l'une des revendications 15 à 17.

## Patentansprüche

1. Wirkstoff-Träger in Form von multilamellaren Vesicula, die aus konzentrischen Membranen bestehen, **dadurch gekennzeichnet, dass** die genannten Membranen mindestens ein nicht-ionisches Tensid vom Saccharoseester-Typ umfassen, der mindestens eine Kette aufweist, die aus einer linearen oder verzweigten, gesättigten oder ungesättigten, gegebenenfalls mono- oder polyhydroxylierten C₁₂-C₂₂-Fettsäure stammt.

2. Träger nach Anspruch 1, **dadurch gekennzeichnet, dass** es sich bei der genannten Fettsäure um eine Fettsäure natürlichen, vorzugsweise pflanzlichen, Ursprungs handelt.

3. Träger nach einem der Ansprüche 1 oder 2, **dadurch gekennzeichnet, dass** der genannte Ester außerdem mindestens eine Carbonsäure aufweist, die aus einer gesättigten oder ungesättigten, hydroxylierten oder nicht hydroxylierten C₂-C₄-Mono- oder -Polysäure stammt, deren Hydroxyl-Funktion(en) frei ist (sind) oder verestert ist (sind) durch eine Carbonsäure wie Essigsäure, Milchsäure, Citronensäure, Weinsäure, Acetylweinsäure, Bernsteinsäure.

4. Träger nach einem der Ansprüche 1 bis 3, **dadurch gekennzeichnet, dass** der genannte Ester im Gemisch mit mindestens einem Mono-, Di- oder Triester von Glycerin und einer linearen oder verzweigten, gegebenenfalls mono- oder polyhydroxylierten C₁₂-C₂₂-Fettsäure vorliegt, wobei das genannte Gemisch als Zuckerglycerid bezeichnet wird.

5. Träger nach einem der Ansprüche 1 bis 4, **dadurch gekennzeichnet, dass** er außerdem mindestens ein weiteres Tensid, z.B. Lecithin, einen Sorbitanester oder ein Polysorbat, enthält.

6. Träger nach einem der Ansprüche 1 bis 5, **dadurch gekennzeichnet, dass** die genannten Fettsäuren unabhängig voneinander ausgewählt sind aus der Gruppe, die besteht aus Laurinsäure, Myristinsäure, Palmitinsäure, Stearinsäure, Behensäure, Ölsäure, Linolsäure, Linolensäure, Arachidonsäure, Rizinolsäure und Mischungen davon.

7. Träger nach einem der Ansprüche 1 bis 6, **dadurch gekennzeichnet, dass** die genannten Vesicula aus einer multilamellaren Anordnung von konzentrischen Doppelschichten aufgebaut sind, die mindestens ein Tensid vom Ester-Typ umfassen und durch ein Medium voneinander getrennt sind, das aus einer polaren Flüssigkeit besteht, die als interstitielles Lösungsmittel bezeichnet wird.

8. Träger nach einem der Ansprüche 1 bis 7, **dadurch gekennzeichnet, dass** die genannten Vesicula Dimensionen zwischen 0,1 und 50 µm, vorzugsweise zwischen 0,2 und 10 µm, haben.

9. Träger nach einem der Ansprüche 1 bis 8, **dadurch gekennzeichnet, dass** die Membranen der genannten Vesicula eine Mischung von mindestens zwei Tensiden umfassen, von denen mindestens eines ein Tensid vom Saccharoseester-Typ ist, wie er in einem der Ansprüche 1 bis 4 definiert ist, wobei die genannten Tenside umfassen ein als lipophiles Agens bezeichnetes erstes Tensid, das ein hydrophiles-lipophiles Gleichgewicht (HLB) zwischen 3 und 7 aufweist, und ein als hydrophiles Agens bezeichnetes zweites Tensid, das ein hydrophiles-lipophiles-Gleichgewicht (HLB) zwischen 8 und 15 aufweist.

10. Träger nach Anspruch 9, **dadurch gekennzeichnet, dass** mindestens eines der Tenside eine kritische Micellen-Konzentration (CMC) von weniger als 10 bis 5 mol/l, vorzugsweise von weniger als 10 bis 6 mol/l, aufweist.

11. Träger nach einem der Ansprüche 9 oder 10, **dadurch gekennzeichnet, dass** das als lipophiles Agens bezeichnete Tensid in Mengenanteilen, ausgedrückt in Gew.-%, bezogen auf die Gesamtmenge der Tenside, zwischen 0 und 100 %, vorzugsweise zwischen 20 und 100 %, vorliegt.

12. Träger nach einem der Ansprüche 1 bis 11, **dadurch gekennzeichnet, dass** die genannten Vesicula mindestens ein Tensid enthalten, das zur Familie der Zuckerester von Fettsäuren gehört, die aus pflanzlichen Fetten stammen.

13. Träger nach einem der Ansprüche 1 bis 12, **dadurch gekennzeichnet, dass** die genannten Vesicula außerdem ein Polymer umfassen, das sie verstärken soll, wobei das genannte Polymer entweder zum Umhüllen der genannten Vesicula verwendet wird oder in ihrem Innern eingekapselt wird.

14. Träger nach Anspruch 13, **dadurch gekennzeichnet, dass** das genannte Polymer ausgewählt ist aus den folgenden Polymerklassen:
- linearen oder substituierten, neutralen oder ionischen Polysacchariden natürlichen oder modifizierten Ursprungs, insbesondere Guargummis, Karubagummis, Gummiarabicum, (Kappa-, Iota- und Lambda-)-Carraghenanen, Xanthangummis, nativen, veresterten oder amidierten Pectinen, Alginsäure und ihren Salzen, Hyaluronsäure, quatemisierten Guargummis, Chitosan und seinen substituierten Derivaten,
- Gelatine,
- wasser- oder fettlöslichen synthetischen Polymeren wie Polyacrylamid, Polyvinylpyrrolidon, Polyethylenglycol.

15. Zusammensetzung für die Verwendung in Nahrungsmitteln oder Lebensmitteln, **dadurch gekennzeichnet, dass** sie mindestens einen Wirkstoff enthält, der in Vesicula eingekapselt ist, die den Träger nach einem der Ansprüche 1 bis 14 aufbauen.

16. Zusammensetzung nach Anspruch 15, **dadurch gekennzeichnet, dass** der genannte Wirkstoff ausgewählt ist aus der Familie, die besteht aus Produkten vom Säure-Typ für die Verwendung in Lebensmitteln, insbesondere Ascorbinsäure oder Milchsäure, Färbemitteln, insbesondere Pigmenten für Fleisch, Lipiden, Vitaminen, Lebensmittelaromastoffen, essentiellen Ölen, Enzymen.

17. Kosmetische oder pharmazeutische, insbesondere dermatologische, Zusammensetzung, **dadurch gekennzeichnet, dass** sie mindestens einen Wirkstoff umfasst, der in Vesicula eingekapselt ist, die den Träger aufbauen, wie er in einem der Ansprüche 1 bis 14 definiert ist.

18. Verwendung von Tensiden, die zur Familie der Zuckerester und Zuckerglyceride gehören, zur Herstellung eines Trägers nach einem der Ansprüche 1 bis 14 oder einer Zusammensetzung nach einem der Ansprüche 15 bis 17.

## Claims

1. An active principle carrier in the form of multilamellar vesicles constituted of concentric membranes, **characterised in that** said membranes comprise at least one non-ionic surfactant of the sucrose ester type comprising at least one chain arising from a linear or branched, saturated or unsaturated, optionally mono- or polyhydroxylated C₁₂ to C₂₂ fatty acid.

2. The carrier according to claim 1, **characterised in that** said fatty acid is a fatty acid of natural origin, preferably of plant origin.

3. The carrier according to one of claims 1 or 2, **characterised in that** said ester further comprises at least one carboxylic chain arising from a saturated or unsaturated, hydroxylated or non-hydroxylated C₂ to C₄ mono- or polyacid, the hydroxyl function(s) being free or esterified by a carboxylic acid, for example acetic acid, lactic acid, citric acid, tartaric acid, acetyltartaric acid, succinic acid.

4. The carrier according to one of claims 1 to 3, **characterised in that** said ester is in a mixture with at least one glycerol mono-, di- or triester and a linear or branched, optionally mono- or polyhydroxylated C₁₂ to C₂₂ fatty acid, said mixture being designated sucroglyceride.

5. The carrier according to one of claims 1 to 4, **characterised in that** it further contains at least one other surfactant, for example lecithin, a sorbitan ester or a polysorbate.

6. The carrier according to one of claims 1 to 5, **characterised in that** said fatty acids are selected independently from the group consisting of lauric acid, myristic acid, palmitic acid, stearic acid, behenic acid, oleic acid, linoleic acid, linolenic acid, arachidonic acid, ricinoleic acid and their mixtures.

7. The carrier according to one of claims 1 to 6, **characterised in that** said vesicles are constituted of a multilamellar arrangement of concentric bi-layers comprising at least one ester-type surfactant and separated by a medium constituted of a polar liquid known as interstitial solvent.

8. The carrier according to one of claims 1 to 7, **characterised in that** said vesicles have dimensions between 0.1 and 50 µm, preferably between 0.2 and 10 µm.

9. The carrier according to one of claims 1 to 8, **characterised in that** the membranes of said vesicles comprise a mixture of at least two surfactants at least one of which is a surfactant of the sucrose ester type as defined in one of claims 1 to 4, said surfactants comprising a first surfactant, known as lipophilic agent, having a hydrophilic-lipophilic balance (HLB) between 3 and 7 and a second surfactant agent, known as hydrophilic agent, having an HLB between 8 and 15.

10. The carrier according to claim 9, **characterised in that** at least one of the surfactant agents has a critical micellar concentration (CMC) lower than 10⁻⁵ mol/l, preferably lower than 10⁻⁶ mol/l.

11. The carrier according to one of claims 9 or 10, **characterised in that** the surfactant agent known as lipophilic agent is present in proportions expressed in percentages by weight with respect to the whole of the surfactants between 0 and 100 %, preferably between 20 and 100 %.

12. The carrier according to one of claims 1 to 11, **characterised in that** said vesicles contain at least one surfactant belonging to the family of fatty acid sucroesters derived from vegetable fats.

13. The carrier according to one of claims 1 to 12, **characterised in that** said vesicles further comprise a polymer intended for reinforcing them, said polymer being either used for coating said vesicles, or encapsulated within them.

14. The carrier according to claim 13, **characterised in that** said polymer is selected from the following classes of polymers :
- natural or modified, linear or substituted, neutral or ionic polysaccharides, in particular guar gums, locust bean gums, gum arabic, carrageenans (kappa, iota and lambda), xanthan gums, natural, esterified or amidated pectins, alginic acid and its salts, hyaluronic acid, quaternised guar gums, chitosan and its substituted derivatives,
- gelatine,
- hydro or liposoluble synthetic polymers such as polyacrylamide, polyvinylpyrrolidone, poly(ethylene glycol).

15. A composition for use as food or diet food, **characterised in that** it contains at least one active product encapsulated in the vesicles constituting the carrier according to one of claims 1 to 14.

16. The composition according to claim 15, **characterised in that** said active product is selected from the family consisting of acid-type products for use as food, notably ascorbic acid or lactic acid, colouring agents, in particular pigments for meat, lipids, vitamins, food flavours, essential oils, enzymes.

17. The cosmetic or pharmaceutical composition, notably dermatological composition, **characterised in that** it comprises at least one active principle encapsulated in the vesicles constituting the carrier as defined according to one of claims 1 to 14.

18. Use of surfactants belonging to the family of sucroesters and sucroglycerides for the manufacture of a carrier according to one of claims 1 to 14, or of a composition according to one of claims 15 to 17.
